# EUROPÄISCHE PATENTSCHRIFT

(11) **EP 0 487 830 B1**
(45) Veröffentlichungstag und Bekanntmachung des Hinweises auf die Patenterteilung: **15.06.1994**
(21) Anmeldenummer: 91113106.8
(22) Anmeldetag: 05.08.1991
(51) Int. Cl.: A61B 17/58

(54) **Korrekturimplantat für die menschliche Wirbelsäule**
Implant for correction of the humain spinal column
Implant de correction du rachis humain

(30) Priorität: 29.11.1990 DE 9016227 U
(43) Veröffentlichungstag der Anmeldung: 03.06.1992
(73) Patentinhaber: Howmedica GmbH, D-24232 Schönkirchen (DE)
(72) Erfinder: Westermann, Kord, Dr. med., W-3000 Hannover 61 (DE)
(74) Vertreter: Patentanwälte Hauck, Graalfs, Wehnert, Döring, Siemons

(56) Entgegenhaltungen:
- EP-A- 0 383 992
- WO-A-90/00377
- FR-A- 2 615 095
- FR-A- 2 624 720

## Beschreibung

Die Neuerung bezieht sich auf ein Korrekturimplantat für die menschliche Wirbelsäule nach dem Oberbegriff des Anspruchs 1.

Aus der DE-A- 26 49 042 ist ein Implantat zur ventralen Derotationsspondylodese bekanntgeworden, bei dem Pedikelschrauben zum Einschrauben in die Wirbelspongiosa am Kopf einen offenen Schlitz aufweisen zur Aufnahme eines einlegbaren Kompressionsstabes. Der Kompressionsstab wird beidseits jedes Schraubenkopfs mit Hilfe einer Mutter auf dem Kompressionsstab verspannt. Unterhalb des Schraubenkopfes befindet sich eine Druckverteilungsplatte, die an die Form des Wirbelkörpers angepaßt ist.Das bekannte Implantat wird häufig paarweise eingesetzt auf beiden Seiten der Wirbelkörper, wobei der Kompressionsstab durch Biegung an die Krümmung der Wirbelsäule im betreffenden Bereich angepaßt wird. Die Stabilität des Kompressionsstabes ist verhältnismäßig gering. Eine Querverspannung zwischen paarweise angeordneten Implantaten ist nicht vorgesehen.

Aus der DE-A- 32 19 575 ist ein Korrekturimplantat für fehlgeformte Wirbelsäulen unter Verwendung von Knochenschrauben bekanntgeworden, bei dem die Knochenschrauben mit Eckstücken versehen sind zwecks Verbindung mit einem sogenannten Spannschloß. Mit Hilfe des Spannschlosses läßt sich der Abstand der Pedikelschrauben variieren. Paarweise eingesetzte Implantate dieser Art können durch kreuzweise verspannte Dräht auch gegeneinander stabilisiert werden. Drähte können jedoch bekanntlich nur auf Zug beansprucht werden, nicht auf Druck und auf Torsion. Nachteilig beim bekannten Implantat ist ferner, daß die Schrauben aus unterschiedlichen Richtungen betätigt werden müssen.

Aus der DE-A- 38 23 737 ist eine gattungsgemäße Korrektur- und Haltevorrichtung für die menschliche Wirbelsäule bekanntgeworden, bei der die Pedikelschrauben gelenkig mit Aufnahmeteilen verbunden sind, die ihrerseits mit einem ersten Paar von Gewindestangen und einem zweiten Paar von Gewindestangen verbindbar sind. Das erste Paar Gewindestangen verbindet die Pedikelschrauben auf einer Seite der Wirbelsäule, während das zweite Paar der Gewindestangen sich überkreuzt. Der technische Aufbau des Korrekturimplantats ist verhältnismäßig aufwendig. Die Betätigung der Schrauben muß aus unterschiedlichen Richtungen erfolgen, was für den Chirurgen eine Erschwernis bedeutet.

Der Neuerung liegt die Aufgabe zugrunde, ein Korrekturimplantat für die menschliche Wirbelsäule zu schaffen, das einfach aufgebaut ist und aus wenigen Teilen besteht und das ferner einfach angebracht werden kann und die reponierten Wirbelkörper in beliebiger Lage wirksam fixiert.

Diese Aufgabe wird erfindungsgemäß gelöst durch die Merkmale des Kennzeichnungsteils des Anspruchs 1.

Beim neuerungsgemäßen Korrekturimplantat weisen die Pedikelschrauben in an sich bekannter Weise einen Aufnahmeschlitz auf zur Aufnahme von Stangen. Diese dienen jedoch dazu, Pedikelschraubenpaare kreuzweise zu verspannen. Die Längsverbindung zwischen den Pedikelschrauben erfolgt mit Hilfe plattenförmiger länglicher Elemente, die Löcher zum Hindurchstecken von Pedikelschrauben aufweisen, wobei mindestens ein Loch als Langloch ausgeführt ist, damit eine Pedikelschraube unabhängig von ihrer Lage zum plattenförmigen Element in richtiger Position in den Pedikel eingeschraubt werden kann. Die Pedikelschrauben weisen im oberen Bereich eine Schulter auf, die sich gegen die zugekehrte Seite des plattenförmigen Elements anlegt.

Zur Verspannung des Stabes an der Pedikelschraube kann dieser mit einem Gewinde versehen werden zur Aufnahme von zwei Muttern, die von entgegengesetzten Seiten mit dem Kopf der Pedikelschraube in Eingriff treten. Eine Ausgestaltung der Neuerung sieht indessen vor, daß der Kopf der Pedikelschraube ein Außengewinde aufweist, auf das eine Mutter schraubbar ist. Die Mutter kann sich von oben gegen den Stab legen und diesen im Schlitz des Kopfes fixieren, wobei die Mutter verhindert, daß die den Aufnahmeschlitz bildenden Schenkel auseinanderbiegen. Zusätzlich oder alternativ kann der Schlitz der Pedikelschraube ein Innengewinde aufweisen, in das eine Feststellschraube einschraubbar ist. Um die Stange besser fixieren zu können, kann sie mit einer Rillung, vorzugsweise einem Gewinde, versehen werden, wodurch die Stange besser erfaßt werden kann. Außerdem kann nach einer weiteren Ausgestaltung der Erfindung die Stange im Querschnitt unrund sein, vorzugsweise dreieckig und in einem entsprechend geformten Aufnahmeschlitz aufgenommen werden. Dadurch wird die Stange an einer Drehung im Kopf der Pedikelschraube wirksam gehindert, so daß erhebliche Drehkräfte über die Stange übertragen werden. Dadurch wird die Stabilisierungswirkung des neuerungsgemäßen Implantates verbessert.

Nach einer anderen Ausgestaltung der Neuerung weist die Wand des Langloches des plattenförmigen Elements eine Reihe von kreisförmigen, einander paarweise gegenüberliegenden Vertiefungen auf, und der Schaft der Pedikelschrauben unterhalb der Schulter und die Vertiefungen sind so bemessen, daß eine Relativverschiebung von Pedikelschraube und Plattenelement unterbunden ist, wenn die Schulter am Plattenelement anliegt, jedoch möglich ist, wenn die Schulter sich im Abstand zum Plattenelement befindet. Wie bereits erwähnt, kann die Pedikelschraube an einer beliebigen Position relativ zum Plattenelement in einen Pedikel eingeschraubt werden. Wird die Schraube anschließend festgezogen und legt sich die Schulter gegen die Platte, haben Platte und Pedikelschraube eine feste Lage zueinander, so daß auch die Platte nunmehr Kräfte zur Fixierung des Implantats und Stabilisierung der Wirbelkörper übernehmen kann.

Die Neuerung wird nachfolgend anhand von Zeichnungen näher erläutert.
- Fig. 1: zeigt die Draufsicht auf ein Korrekturimplantat nach der Neuerung.
- Fig. 2: zeigt vergrößert eine Feststellschraube für das Implantat nach Fig. 1.
- Fig. 3: zeigt einen Schnitt durch eine Stange des Korrekturimplantats nach Fig. 1 entlang der Linie 3-3.
- Fig. 4: zeigt eine Draufsicht auf eine Platte des Korrekturimplantats nach Fig. 1.
- Fig. 5: zeigt einen Schnitt durch die Darstellung nach Fig. 4 entlang der Linie 5-5.
- Fig. 6: zeigt vergrößert eine Pedikelschraube teilweise im Schnitt des Korrekturimplantats nach Fig. 1.
- Fig. 7: zeigt eine Seitenansicht des Kopfes der Pedikelschraube nach Fig. 6 in Richtung Pfeil 7.
- Fig. 8: zeigt einen Schnitt durch die Darstellung nach Fig. 7 entlang der Linie 8-8.
- Fig. 9: zeigt eine Draufsicht einer Mutter des Korrekturimplantats nach Fig. 1.
- Fig. 10: zeigt eine Seitenansicht der Mutter nach Fig. 9.

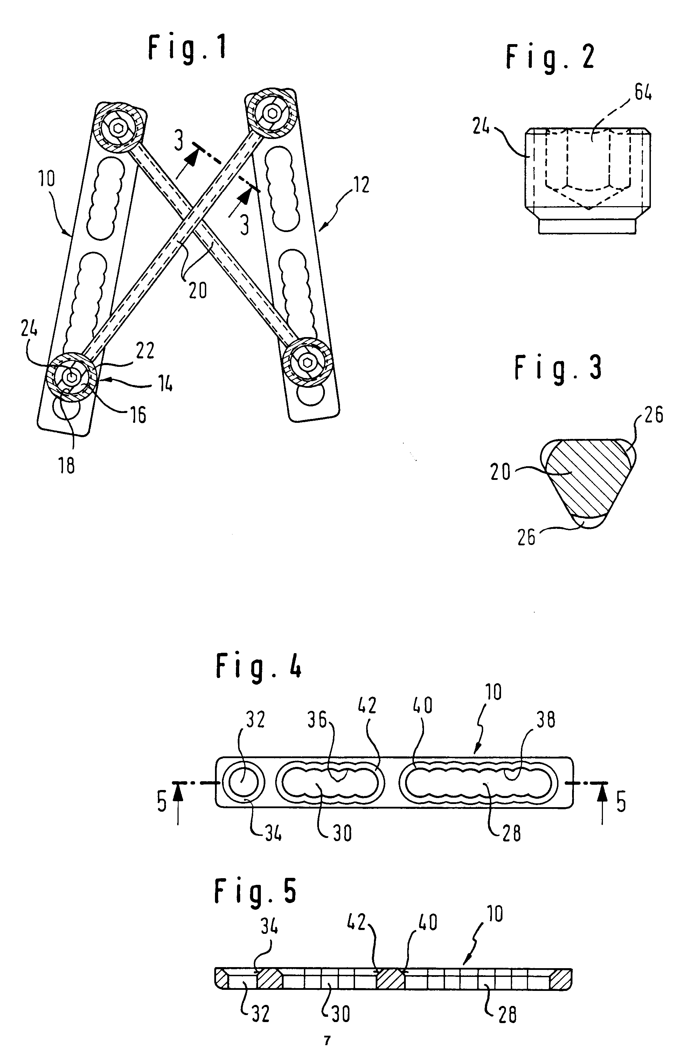

Das in Fig. 1 dargestellte Korrekturimplantat weist zwei Plattenelemente 10, 12 auf. Sie wirken zusammen mit Pedikelschrauben 14, von denen in Fig. 1 nur der Kopf 16 zu erkennen ist. Die Pedikelschrauben haben einen Aufnahmeschlitz 18 für eine Gewindestange 20. In Fig. 1 sind vier Pedikelschrauben kreuzweise mittels der Gewindestangen 20 verbunden. Auf den Kopf 16 der Pedikelschrauben 14 ist eine Mutter 22 geschraubt. Der Kopf 16 weist daher ein entsprechendes Außengewinde auf. Der Schlitz 18 der Pedikelschrauben 14 weist ein Innengewinde auf, in das eine Feststellschraube 24 eingeschraubt werden kann. Nachfolgend werden die in Fig. 1 teilweise nur unzureichend zu erkennenden Teile anhand der nachfolgenden Figuren näher erläutert.

Aus Fig. 3 erkennt man, daß die Stangen 20 im Querschnitt annähernd dreieckig sind, so daß das Gewinde 26 nur teilweise in den Spitzenbereichen geformt ist. Gleichwohl läßt sich eine Mutter auf die Stange 20 schrauben, falls erforderlich.

Aus den Figuren 4 und 5 ist zu erkennen, daß die im Querschnitt gebogene oder gewölbte Platte 10 zwei Langlöcher 28, 30 und ein Kreisloch 32 aufweist. Das Kreisloch 32 besitzt auf der einen Seite eine Ansenkung 34. Die Langlöcher 28, 30 weisen kreisbogenförmige Vertiefungen 36 bzw. 38 auf. Im Bereich der Vertiefungen ist die rund um die Langlöcher 28, 30 vorgenommene Ansenkung 40, 42 sphärisch bzw. ballig.

Die Pedikelschraube 14 weist einen Kopf 16, einen sich an den Kopf anschließenden konischen Abschnitt 46 sowie einen Gewindeschaftabschnitt 48 auf. Das Gewinde des Gewindeschaftabschnitts 48 ist zum Beispiel selbstschneidend.

Die Unterseite des Kopfes 16 weist eine ballige Schulter 50 auf. Im oberen Bereich weist der Kopf 16 ein Außengewinde 52 auf, und der Schlitz 18 weist im oberen Bereich ein Innengewinde 54 auf. Im unteren Bereich ist der Schlitz im Querschnitt dreieckförmig und an die Querschnittskontur der Gewindestange 20 angepaßt. An zwei gegenüberliegenden Seiten ist der Kopf 16 mit einer Einsenkung versehen, wie bei 56 dargestellt. Diese Einsenkung kann zum Beispiel dazu dienen, eine Mutter aufzunehmen, die auf die Stange 20 aufgeschraubt ist. Eine Querbohrung 58 dient zur Fixierung des Werkzeugs zum Eindrehen der Pedikelschraube 14.

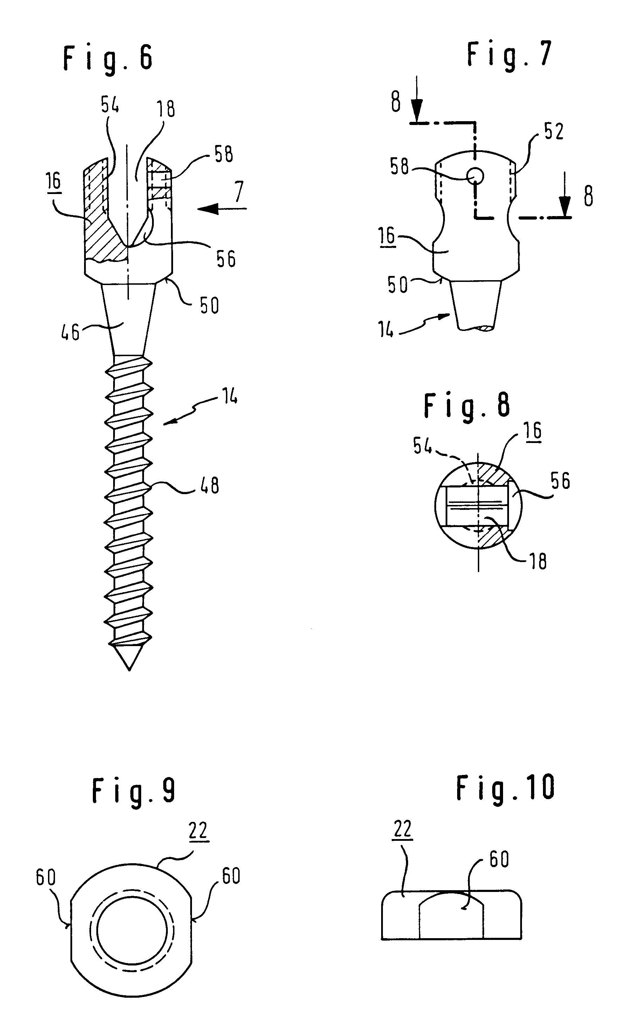

In den Figuren 9 und 10 ist die Mutter 22 zu erkennen, die auf das Gewinde 52 des Kopfes 16 der Pedikelschraube 14 aufgeschraubt werden kann. Sie weist an gegenüberliegenden Seiten Abflachungen 60 für ein Schraubwerkzeug auf.

Bei der Anbringung des Implantates nach Fig. 1 werden zunächst die Pedikelschrauben 14 in die Pedikel der Wirbelkörper eingeschraubt. Sie erstrecken sich dabei durch die entsprechenden Löcher in den Platten 10, 12. Vorzugsweise wird eine Pedikelschraube durch ein Kreisloch 32 hindurchgesteckt, während die andere durch eines der beiden Langlöcher 28, 30 gesteckt wird. Anschließend erfolgt mit Hilfe einer nicht gezeigten Vorrichtung das Reponieren der Wirbelkörper. Ist dieser Vorgang beendet, werden die Pedikelschrauben fest gegen die Platten 10, 12 gepreßt, um die Platten 10, 12 relativ zu den Pedikelschrauben 14 festzulegen. Danach werden die Stangen 20 in die Schlitze 18 der Pedikelschrauben 14 eingelegt und mit Hilfe der Feststellschrauben 24 festgelegt. Wie in Fig. 2 dargestellt, haben die Feststellschrauben 24 ein Außengewinde, das mit dem Innengewinde im Schlitz 18 des Pedikelschraubenkopfes zusammenwirkt sowie einen Innensechskant 64. Schließlich wird eine Mutter 22 auf den Kopf der Pedikelschraube geschraubt, damit die Feststellschraube sicher im zugehörigen Gewinde verbleibt.

## Patentansprüche

1. Korrekturimplantat für die menschliche Wirbelsäule, mit Pedikelschrauben (14) zum Einschrauben in Wirbelkörper, mit einem Paar von Stangen (20), die sich überkreuzend mit den Köpfen von jeweils zwei Pedikelschrauben (14) verbindbar sind, und einem Paar länglicher nebeneinander angeordneter Elemente (10,12), die ebenfalls mit den Pedikelschrauben (14) verbindbar sind, dadurch gekennzeichnet, daß die Köpfe (16) der Pedikelschrauben (14) einen offenen Schlitz (18) aufweisen zur Aufnahme der Stangen (20), die darin mit Befestigungsmitteln (24) verspannbar sind, daß plattenförmige längliche Elemente (10, 12) vorgesehen sind, die Löcher (28, 30, 32) aufweisen, durch die Pedikelschrauben (14) hindurchgeführt sind, wobei mindestens ein Loch (28, 30) als Langloch ausgeführt ist und daß die Pedikelschrauben (14) im oberen Bereich eine Schulter (50) aufweisen, die mit der zugekehrten Seite der plattenförmigen Elemente (10, 12) in Anlage bringbar ist.

2. Implantat nach Anspruch 1, dadurch gekennzeichnet, daß der Kopf (16) der Pedikelschrauben (14) ein Außengewinde (52) aufweist, auf das eine Mutter (22) schraubbar ist.

3. Implantat nach Anspruch 1 oder 2,dadurch gekennzeichnet, daß der Schlitz (18) des Pedikelschraubenkopfes (16) ein Innengewinde (54) aufweist, in das eine Feststellschraube (24) schraubbar ist.

4. Implantat nach einem der Ansprüche 1 bis 3, dadurch gekennzeichnet, daß die Stangen (20) im Querschnitt unrund sind.

5. Implantat nach Anspruch 4, dadurch gekennzeichnet, daß der Schlitz (18) im Grund einen Querschnitt hat, der dem Querschnitt der Stange (20) angepaßt ist.

6. Implantat nach Anspruch 4 und 5, dadurch gekennzeichnet, daß die Stangen (20) und der Schlitzgrund im Querschnitt dreieckig sind.

7. Implantat nach einem der Ansprüche 3 bis 6, dadurch gekennzeichnet, daß die Stange (20) gerillt ist, vorzugsweise ein Gewinde (26) aufweist.

8. Implantat nach einem der Ansprüche 1 bis 7, dadurch gekennzeichnet, daß zwischen dem Gewinde (48) der Pedikelschraube (14) und der Schulter (50) ein konischer Abschnitt (46) vorgesehen ist.

9. Implantat nach einem der Ansprüche 1 bis 8,dadurch gekennzeichnet, daß die Schulter (50) ballig geformt ist und die Löcher (28, 30) in den Plattenelementen (10, 12) eine Fase (40, 42) auf der der Schulter (50) zugewandten Seite aufweisen.

10. Implantat nach einem der Ansprüche 1 bis 9, dadurch gekennzeichnet, daß die Wand des Langlochs (28, 30) eine Reihe von kreisförmigen einander paarweise gegenüberliegenden Vertiefungen (36, 38) aufweist und der Schaft (46, 48) der Pedikelschrauben (40) unterhalb der Schulter (50) und die Vertiefungen (36, 38) so bemessen sind, daß eine Relativverschiebung von Pedikelschraube (14) und Plattenelement (10, 12) unterbunden ist, wenn die Schulter (50) am Plattenelement (10, 12) anliegt, jedoch möglich ist, wenn die Schulter (50) sich im Abstand zum Plattenelement (10, 12) befindet.

11. Implantat nach Anspruch 9 und 10, dadurch gekennzeichnet, daß ballige Ansenkungen im Bereich der Vertiefungen (36, 38) geformt sind.

## Claims

1. A correcting implant for the human spinal column comprising pedicle screws (14) to be fastened to vertebrae, a pair of rods (20) which can be cross-wise connected to the heads of two pedicle screws (14) each, and a pair of elongated elements (10,12) which are arranged side-by-side, which elements can be connected alike to said pedicle screws (14), characterized in that the heads (16) of the pedicle screws (14) are provided with an open slot (18) for receiving said rods (20) which are anchored therein by fastening means (24), that plate-like elongated elements (10,12) are provided which include openings (28,30,32) through which the pedicle screws (14) extend, wherein at least an opening (28,30) is formed as an elongated opening, and that the pedicle screws (14) comprise a shoulder (50) at its upper portion which can be brought into engagement with the adjacent side of the plate-like elements (10,12).

2. Implant of claim 1, wherein the head (16) of the pedicle screws (14) comprises an outer thread (52) onto which a nut (22) can be screwed.

3. Implant of claim 1 or 2, wherein the slot (18) of the pedicle screw head (16) comprises an inner thread (54) into which a set screw (24) can be screwed.

4. Implant of one of claims 1 to 3, wherein the rods (20) are non-circular in cross-section.

5. Implant of claim 4, wherein the slot (18) has a cross-section at the bottom which fits to the cross section of the rod (20).

6. Implant of claim 4 and 5, wherein the rods (20) and the bottom of the slot are triangular in cross-section.

7. Implant of one of claims 3 to 6, wherein the rod (20) is grooved, preferably comprises a thread (26).

8. Implant of one of claims 1 to 7, wherein a tapered section (46) is provided between the thread (48) of the pedicle screw (14) and the shoulder (50).

9. Implant of one of claims 1 to 8, wherein the shoulder (50) is rounded and the openings (28,30) in the plate elements (10,12) comprise a chamfer (40,42) at the side facing the shoulder (50).

10. Implant of one of claims 1 to 9, wherein the wall of the elongated opening (28,30) comprises a number of circular indentations (36,38) which oppose each other in pairs and wherein the shaft (46,48) of the pedicle screws (40) below the shoulder (50) and the indentations (36,38) are selected such that a relative displacement of the pedicle screw (14) and the plate element (10,12) is prevented, when the shoulder (50) engages the plate element (10,12) but is allowed when the shoulder (50) is located in a distance from said plate element (10,12).

11. Implant of claim 9 and 10, wherein rounded counter-sinks are formed in the area of said indentations (36,38).

## Revendications

1. Implant de correction pour le rachis humain, comportant des vis de pédicule (14) à visser dans les vertèbres, comportant une paire de tiges (20) qui en se croisant peuvent être reliées chacune aux têtes de deux vis de pédicule (14), ainsi qu'une paire d'éléments (10, 12) allongés, juxtaposés, qui peuvent être liés aussi avec les vis (14), caractérisé en ce que les têtes (16) des vis (14) présentent une fente (18) ouverte destinée à loger les tiges (20), qui peuvent être serrées à l'intérieur avec des moyens de fixation (24), en ce que sont prévus des éléments allongés (10, 12) en forme de plaque qui présentent des trous (28, 30, 32) à travers lesquels passent les vis (14), un trou (28, 30) au moins étant un trou allongé, et en ce que les vis (14) présentent dans leur zone supérieure un épaulement (50) qui peut être amené en application avec le côté tourné vers celui-ci des éléments (10, 12) en forme de plaque.

2. Implant selon la revendication 1, caractérisé en ce que la tête (16) des vis de pédicule (14) présente un filetage (52) sur lequel peut être vissé un écrou (22).

3. Implant selon la revendication 1 ou 2, caractérisé en ce que la fente (18) de la tête de vis (16) présente un taraudage (54) dans lequel peut être vissé une vis de blocage (24).

4. Implant selon l'une des revendications 1 à 3, caractérisé en ce que les tiges (20) ont une section transversale non ronde.

5. Implant selon la revendication 4, caractérisé en ce que la fente (18) présente au fond une section transversale qui est adaptée à celle de la tige (20).

6. Implant selon les revendications 4 et 5, caractérisé en ce que les tiges (20) et le fond de la fente ont une section transversale triangulaire.

7. Implant selon l'une des revendications 3 à 6, caractérisé en ce que la tige (20) est rainurée, de préférence présente un filetage (26).

8. Implant selon l'une des revendications 1 à 7, caractérisé en ce qu'entre le filetage (48) de la vis (14) et l'épaulement (50) il est prévu une portion conique (46).

9. Implant selon l'une des revendications 1 à 8, caractérisé en ce que l'épaulement (50) est bombé et les trous (28, 30) pratiqués dans les éléments en plaque (10, 12) présentent un chanfrein (40, 42) sur le côté tourné vers l'épaulement (50).

10. Implant selon l'une des revendications 1 à 9, caractérisé en ce que la paroi du trou allongé (28, 30) présente une série de creux (36, 38) circulaires opposés l'un à l'autre deux par deux et la tige (46, 48) des vis de pédicule (40) sous l'épaulement (50) ainsi que les creux (36, 38) ont des dimensions telles qu'un déplacement relatif de la vis (14) et de l'élément en plaque (10, 12) est interdit, lorsque l'épaulement (50) s'applique contre l'élément en plaque (10, 12), mais est possible lorsque l'épaulement (50) se trouve à une certaine distance de l'élément en plaque (10, 12).

11. Implant selon les revendications 9 et 10, caractérisé en ce que des chanfreins bombés sont formés dans la zone des creux (36, 38).
